(19) Europäisches Patentamt
European Patent Office
Office européen des brevets

(11) Veröffentlichungsnummer: **0 520 242 B1**

# (12) EUROPÄISCHE PATENTSCHRIFT

(45) Veröffentlichungstag der Patentschrift: **19.07.95**

(51) Int. Cl.[6]: **C07C 323/59**, C07C 323/60, C07D 277/06, C08F 20/54

(21) Anmeldenummer: **92109744.0**

(22) Anmeldetag: **10.06.92**

## (54) Optisch aktive schwefelhaltige Aminosäure-Derivate, ihre Herstellung, ihre Polymerisation zu optisch aktiven Polymeren und deren Verwendung.

(30) Priorität: **22.06.91 DE 4120695**

(43) Veröffentlichungstag der Anmeldung:
**30.12.92 Patentblatt 92/53**

(45) Bekanntmachung des Hinweises auf die Patenterteilung:
**19.07.95 Patentblatt 95/29**

(84) Benannte Vertragsstaaten:
**AT BE CH DE DK ES FR GB GR IT LI LU NL PT SE**

(56) Entgegenhaltungen:
**DE-A- 3 706 890**
**US-A- 3 615 624**
**US-A- 3 846 306**
**US-A- 3 884 761**

**CHEMICAL ABSTRACTS, vol. 69, no. 9, 26. August 1968, Columbus, Ohio, US; abstract no. 36430, 'ROTATORY DISPERSION OF OSMATE ESTERS OF OPTICALLY ACTIVE ACRYLOYL ALPHA-AMINO ACID METHYL ESTERS' Seite 3414 ;Spalte 2 ;**

(73) Patentinhaber: **BAYER AG**
**D-51368 Leverkusen (DE)**

(72) Erfinder: **Grosse-Bley, Michael, Dr.**
**Dauner Strasse 7**
**W-5000 Köln 41 (DE)**
Erfinder: **Bömer, Bruno, Dr.**
**Max-Planck-Strasse 53**
**W-5060 Bergisch Gladbach 2 (DE)**
Erfinder: **Grosser, Rolf, Dr.**
**Gellertstrasse 9**
**W-5090 Leverkusen (DE)**
Erfinder: **Arlt, Dieter, Prof.Dr.**
**Rybniker Strasse 2**
**W-5000 Köln 80 (DE)**
Erfinder: **Lange, Walter, Dr.**
**Auerstrasse 7**
**W-5000 Köln 60 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CHEMICAL ABSTRACTS, vol. 99, no. 7, 15. August 1983, Columbus, Ohio, US; abstract no. 53714, 'SYNTHESIS AND STERIC STRUCTURE OF 9-THIA-1,5DIAZABICYCLO[5.3.0]DECANE-2,6-DIONE' Seite 556 ;Spalte 1 ;

CHROMATOGRAPHIA Bd. 27, Nr. 11, Juni 1989, BRAUNSCHWEIG DE Seiten 535 - 543; H. ENGELHARDT ET AL: 'POLYMER ENCAPSULATED STATIONARY PHASES'

**Beschreibung**

Die Erfindung betrifft neue optisch aktive schwefelhaltige Aminosäure-Derivate, ein Verfahren zu ihrer Herstellung, ihre Polymerisation zu optisch aktiven Polymeren und die Verwendung dieser optisch aktiven Polymere als Adsorbentien für die chromatographische Trennung von Racematen in die Enantiomere.

Die Auftrennung von Enantiomerengemischen in die Enantiomere hat in den letzten Jahren immer mehr an Bedeutung gewonnen, weil sich gezeigt hat, daß sich die Enantiomere biologisch wirksamer Racemate stark in ihren Wirkungen und Nebenwirkungen unterscheiden können. Daher besteht ein großes Interesse an der Isolierung der einzelnen Enantiomere biologisch wirksamer Racemate oder Enantiomerengemische.

Es wurden bereits die verschiedensten Adsorbentien für die chromatographische Racemattrennung vorgeschlagen. Als sehr vorteilhafte Adsorptionsmittel haben sich dabei bislang die z.B. in Chem. Ber. 109 (1976), 1967 oder EP-A 379 917 beschriebenen polymeren (Meth)acrylsäure-Derivate optisch aktiver Amino-Verbindungen erwiesen, gegebenenfalls in an anorganischen Trägern immobilisierter Form.

Ähnliche Aminosäurederivate sind bereits bekannt aus US 3 846 306. Daraus erhältliche Polymere werden beschrieben, in denen nach Reduktion $R_2$ Wasserstoff bzw. eine Thioreaktivgruppe bedeutet, die direkt über Schwefel binden und hydrophile Polymere bilden. Im Gegensatz dazu betrifft die vorliegende Erfindung Verbindungen mit inaktiven schwefelhaltigen Gruppen (bisalkylierte/arylierte Sulfide), die nicht direkt über Schwefel binden und hydrophobe, wasserunlösliche Polymere bilden. Auch in US 3 884 761 werden hydrophile Polymere beschrieben, die eine Thiolseitenkette tragen, an welche biologisch aktive Verbindungen gebunden werden können.

US 3 615 625 beschreibt lichtempfindliche silberhaltige Verbindungen, die für photographische Zwecke eingesetzt werden können und gibt somit keinen Hinweis auf den Gegenstand der Anmeldung. In Chem. Abstr., Vol. 69, S. 3414, Nr. 36430d wird das Monomer N-Acryloyl-L-methionin-methylester genannt. Diese Publikation enthält jedoch ebenfalls keinen Hinweis auf den Gegenstand der vorliegenden Anmeldung, insbesondere auf die gewünschte Trennung von racemischen Gemischen.

Es wurde nun überraschend gefunden, daß Aminosäure-Derivate, die sich von schwefelhaltigen Aminosäuren ableiten, in Kombination mit speziellen Ester- und Amidresten, insbesondere mit sterisch anspruchsvollen und starren Ester- bzw. Amid-Resten zu sehr interessanten neuen optisch aktiven Adsorbentien führen. Völlig unerwartet läßt sich feststellen, daß ein Schwefelatom in der Aminosäure-Komponente die Trenneigenschaften für viele Trennprobleme gegenüber einem reinen Kohlenwasserstoff-Rest deutlich verbessert. Auch die Löslichkeit der Monomere wird günstig beeinflußt.

An den erfindungsgemäßen schwefelhaltigen Trennmaterialien können beispielsweise eine Reihe von Wirkstoff-Racematen deutlich besser getrennt werden als an den aus dem Stand der Technik bekannten Adsorbentien.

Die Erfindung betrifft daher optisch aktive schwefelhaltige Aminosäure-Derivate der Formel (I)

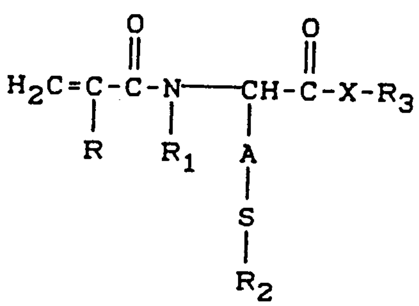

(I),

in welcher

- R für Wasserstoff, Methyl oder Fluor steht,
- $R_1$ Wasserstoff oder Methyl bedeutet oder zusammen mit $R_2$ eine Methylengruppe, die ein- oder zweifach Methyl- oder einfach tertiär -Butyl-substituiert sein kann, oder eine Dimethylengruppe bildet,
- $R_2$ für Alkyl mit bis zu 8 C-Atomen, Phenyl,

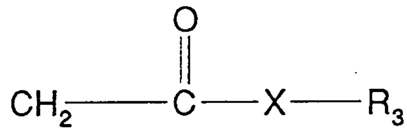

steht oder zusammen mit $R_1$ eine dort beschriebene Brücke bildet,

$R_3$ für einen $C_{10}$-Terpenylrest, einen Adamantylrest oder einen Decahydronaphthylrest steht oder für einen Alkylrest oder Cycloalkylrest mit jeweils bis zu 12 C Atomen mit der Ausnahme von Methyl und Ethyl steht, der gegebenenfalls ein- oder zweifach substituiert ist durch $C_6$-$C_{10}$-Aryl, $C_1$-$C_4$-Alkoxy, $C_3$-$C_{12}$-Cycloalkyl oder Halogen, wobei die genannten Aryl- und Cycloalkylreste ihrerseits wieder durch $C_1$-$C_4$-Alkyl substituiert sein können,

X Sauerstoff oder eine $NR_4$-Gruppe bedeutet, in der $R_4$ für Wasserstoff oder $C_1$-$C_4$-Alkyl steht oder zusammen mit $R_3$ einen stickstoffhaltigen 5- bis 7-gliedrigen Ring bildet, der gegebenenfalls ein- oder zweifach $C_1$-$C_4$-alkyl- oder $C_1$-$C_6$-alkoxycarbonylsubstituiert sein kann,

A für eine Methylen- oder eine Dimethylengruppe steht.

X Sauerstoff oder eine $NR_4$-Gruppe bedeutet, in der $R_4$ für Wasserstoff oder $C_1$-$C_4$-Alkyl steht oder zusammen mit $R_3$ einen stickstoffhaltigen 5- bis 7-gliedrigen Ring bildet, der ein- oder zweifach $C_1$-$C_4$-alkyl- oder $C_1$-$C_6$-alkoxycarbonylsubstituiert sein kann,

A für eine gegebenenfalls ein- oder zweifach $C_1$-$C_4$-alkylsubstituierte Methylen- oder Dimethylen-Gruppe steht.

Bevorzugte Verbindungen der Formel (I) sind die in den Beispielen 1 - 25 genannten Stoffe.

Die optisch aktiven Aminosäure-Derivate leiten sich vorzugsweise von optisch aktiven schwefelhaltigen Aminosäuren wie z.B. Cystein, Penicillamin, oder Homocystein ab Von besonderem Interesse sind dafür Verbindungen deren SH-Funktion alkyliert ist z.B. S-Methyl-cystein, S-Benzyl-cystein, S-Methyl-penicillamin, Methionin Ethionin oder Butionin, oder acyliert ist z.B. S-Acetylcystein oder Alkoxycarbonylmethyliert ist z.B. S-Menthoxycarbonylmethyl-cystein oder über eine Alkylenbrücke mit der Aminogruppe verbunden ist z.B. Thioprolin, 2,2-Dimethyl-thioprolin, 5,5-Dimethyl-thioprolin oder 2-tert.-Butylthioprolin.

Für $R_3$ ist die Verwendung optisch aktiver Reste besonders vorteilhaft, z.B. des 1-Phenylethyl-, l-(1-Naphthylethyl)-, l-(2-Naphthylethyl)-, des d- oder l-Menthyl, d- oder l-Neomenthyl-, d- oder l-Bornyl-, d-oder l-Fenchyl- oder des d- oder l-Pinanylrestes.

Die erfindungsgemäßen optisch aktiven schwefelhaltigen Aminosäurederivate der Formel (I) werden erhalten durch Umsetzung von optisch aktiven schwefelhaltigen Aminosäurederivaten der Formel (II)

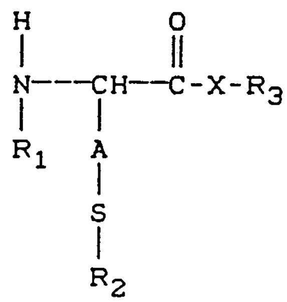

(II),

in der

$R_1$, $R_2$, $R_3$, A und X die unter Formel (I) angegebene Bedeutung haben,

oder deren Säureadditionsprodukten mit Acryloyl-Derivaten der Formel (III)

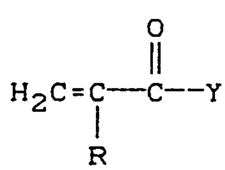

(III),

in der

R die unter Formel (I) angegebene Bedeutung hat und

Y für Fluor, Chlor oder Brom steht oder für den Rest

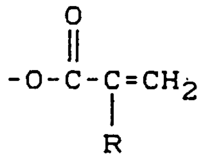

steht (Anhydrid),

gegebenenfalls in Gegenwart eines Säurebindemittels in inerten organischen Lösungsmitteln.

Die als Ausgangsverbindungen verwendeten optisch aktiven schwefelhaltigen Aminosäurederivate der Formel (II) sind bekannt oder können nach an sich bekannten Verfahren hergestellt werden (siehe Bull. Chem. Soc. Jpn. 37 (1964), 191).

Geeignete Säureadditionsverbindungen der als Ausgangsstoffe zu verwendenden schwefelhaltigen Aminosäuren sind Salze dieser Aminosäuren mit anorganischen oder organischen Säuren. Bevorzugt sind Mineralsäuren wie Salzsäure, Bromwasserstoffsäure, Schwefelsäure, Phosphorsäure oder organische Säuren wie Essigsäure, Trifluoressigsäure, Methan-, Ethan-, Benzol- oder Toluolsulfonsäure.

Als Lösungsmittel eignen sich alle unter den Reaktionsbedingungen inerten organischen Lösungsmittel. Bevorzugt sind Kohlenwasserstoffe wie Toluol, Petrolether oder Halogenkohlenwasserstoffe wie Dichlormethan, Dichlorethan oder Trichlorethylen oder Ether wie tert.-Butylmethylether.

Als Säurebindemittel kommen vor allem die üblichen anorganischen oder organischen Basen in Betracht; bevorzugt werden Alkali- oder Erdalkalihydroxide wie Natrium-, Kalium-, Lithium-, Calcium- oder Bariumhydroxid, Alkali- oder Erdalkalicarbonate wie Natrium- oder Kaliumcarbonat oder Natriumbicarbonat, Alkalialkoholate wie Natriummethylat, -ethylat, Kaliummethylat, -ethylat, tert.-butylat oder Amine wie Triethylamin oder Pyridin verwendet.

Die Umsetzung der Acryloyl-Derivate der Formel (III) mit den schwefelhaltigen Aminosäure-Derivaten der Formel (II) wird bevorzugt bei Temperaturen von -78 bis +100 °C, insbesondere von -20 bis +60 °C vorgenommen.

Die Erfindung betrifft auch die durch Polymerisation bzw. Copolymerisation der optisch aktiven schwefelhaltigen Acryloylaminosäure-Derivate der Formel (I) erhältlichen optisch aktiven Polymere und Copolymere, die mindestens 40 Mol-%, vorzugsweise mindestens 50 Mol-% Struktureinheiten der Formel (IV)

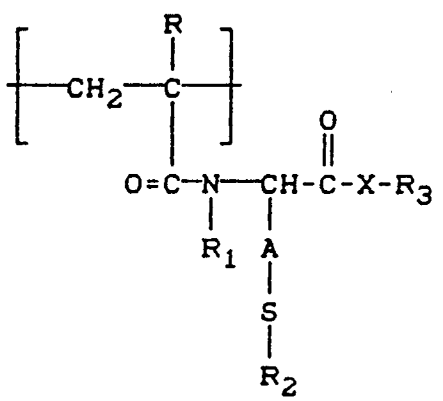

(IV)

enthalten, in der

R, $R_1$, $R_2$, $R_3$, A und X die unter Formel (I) angegebene Bedeutung haben.

Vorzugsweise liegen die erfindungsgemäßen optisch aktiven Polymere der Formel (IV) in Form von vernetzten unlöslichen aber quellbaren Perlpolymerisaten oder in an feinteilige anorganische Trägermaterialien wie z.B. Kieselgel gebundener Form vor. Sie können auch als lineare, in geeigneten organischen Lösungsmitteln lösliche Polymere hergestellt werden. Es ist ferner möglich, verschiedene erfindungsgemäße schwefelhaltige Acryloylaminosäure-Derivate der Formel (I) cozupolymerisieren, sowie 0,1 bis 60, vorzugsweise 0,1 bis 20 Mol-% copolymerisierbarer, anderer Monomere in die Polymere einzubauen.

Die vernetzten Polymerisate liegen vorzugsweise in Form kleiner Teilchen (Perlen) mit 5 bis 200 µm Teilchendurchmesser vor. Sie werden z.B. durch Suspensionspolymerisation der optisch aktiven schwefelhaltigen Acryloylaminosäure-Derivate der Formel (I) mit 0,5 bis 50 Mol-%, vorzugsweise 1 bis 30 Mol-%, besonders bevorzugt 3 bis 20 Mol-% (bezogen auf die Gesamtmenge [Mol] der eingesetzten Monomere) eines geeigneten Vernetzers in an sich bekannter Weise hergestellt.

Durch die Art und Menge der Vernetzer läßt sich der Quellungsgrad der (Perl)Polymerisate nach üblichen Methoden einstellen.

Bei der praktischen Verwendung haben sich (Perl)Polymerisate mit einem Quellungsgrad (Q) von 1.1 bis 12.0, bevorzugt von 2.0 bis 8.0 bewährt.

Der Quellungsgrad Q wird wie folgt bestimmt:

$$Q = \frac{\text{Polymerisationsvolumen (gequollen)}}{\text{Polymerisationsvolumen (ungequollen)}}$$

Als Vernetzungsmittel kommen Verbindungen in Frage, die mindestens zwei polymerisierbare Vinylgruppen enthalten, Bevorzugte Vernetzungsmittel sind Alkandioldiacrylate wie 1,6-Hexandioldiacrylat, 1,4-Butandioldiacrylat, 1,3-Propandioldiacrylat oder 1,2-Ethylenglykoldiacrylat, oder Alkandioldimethacrylate wie 1,4-, 1,3- oder 2,3-Butandioldimethacrylat, 1,3-Propandioldimethacrylat oder 1,2-Ethylenglykoldimethacrylat, aromatische Divinylverbindungen wie beispielsweise Divinylbenzol, Divinylchlorbenzol oder Divinyltoluol, Dicarbonsauredivinylester wie Adipinsäuredivinylester, Benzoldicarbonsäuredivinylester, Terephthalsäuredivinylester, N,N'-Alkylendiacrylamide wie N,N'-Methylendiacrylamid, N,N'-Ethylendiacrylamid, N,N'-Methylendimethacrylamid, N,N'-Ethylendimethacrylamid oder N,N'-Dimethyl-ethylendiacrylamid.

Als Radikalbildner kommen die üblichen Radikalbildner in Frage. Bevorzugt sind Peroxide wie beispielsweise Dibenzoylperoxid, Dilauroylperoxid oder Di-orthotolylperoxid, Perester wie tert.-Butylperpivalat oder tert.-Butylperoctanoat, oder Azoverbindungen wie beispielsweise Azobisisobuttersäurenitril (AIBN). Auch Gemische verschiedener Radikalbildner sind verwendbar.

Die Polymerisationskomponenten werden in einem nicht mit Wasser mischbaren organischen Lösungsmittel, vorzugsweise einem aliphatischen oder aromatischen Kohlenwasserstoff wie Hexan, Heptan, Isodecan, Benzol oder Toluol, einem Halogenkohlenwasserstoff wie Di-, Tri-, Tetrachlormethan oder 1,2-Dichlorethan, einem Ester wie Essigsäureethylester, Essigsäurebutylester oder Dialkylcarbonaten oder einem nicht wasserlöslichen Keton wie Methylisobutylketon oder Cyclohexanon gelöst.

Die organische Phase wird mit Hilfe eines wirksamen Rührers in der wäßrigen Lösung eines Schutzkolloids, bevorzugt in einer wäßrigen Lösung von Polyvinylalkohol, Polyvinylpyrrolidon oder eines Copolymers aus Methacrylsäure und Methylmethacrylat, gleichmäßig verteilt. Je Gewichtsteil organische Phase verwendet man etwa 1 bis 20, bevorzugt 2 bis 10 Gew.-Teile wäßrige Phase. Das Polymerisationsgemisch wird unter Rühren in einer Inertgasatmosphäre, vorzugsweise unter Stickstoff, auf Temperaturen von 30 °C bis 100 °C, vorzugsweise 40 °C bis 80 °C erhitzt. Die Polymerisationsdauer beträgt zwischen 2 und 24, bevorzugt 4 und 12 Stunden. Das auf diese Weise erhaltene Copolymerisat wird durch Filtration von der flüssigen Phase getrennt, durch gründliches Waschen mit Wasser und mit organischen Lösungsmitteln wie Methanol, Ethanol, Benzol, Toluol, Di-, Trichlormethan oder Aceton gereinigt und anschließend getrocknet.

Insbesondere für analytische Anwendungen werden die erfindungsgemäßen optisch aktiven Polymere vorzugsweise in an feinteilige anorganische Träger gebundener Form eingesetzt. Die Herstellung solcher optisch aktiver Chromatographiephasen kann beispielsweise nach den in DE-A 3 706 890 beschriebenen Verfahren erfolgen.

Bevorzugt ist die Polymerisation der optisch aktiven schwefelhaltigen Aminosäure-Derivate der Formel (I) in Gegenwart von Kieselgel-Vinylphasen, die nach B.B. Wheals, J. Chromatogr. 107 (1975), 402 und H. Engelhardt et al., Chromatographia 27 (1989), 535 erhältlich sind, oder von Kieselgel-Diolphasen, die mit (Meth)acrylsäure verestert wurden. Die Polymerisation kann dabei in Abwesenheit von Lösungsmitteln oder in Gegenwart von Lösungsmitteln oder von Fällungsmitteln für die schwefelhaltigen Poly-N-Acryloylamid-Derivate durchgeführt werden. Als Initiatoren können die zur Herstellung der Perlpolymerisate verwendeten Radikalbildner ebenfalls eingesetzt werden.

Die polymermodifizierten Kieselgele enthalten vorzugsweise 1 bis 40 Gew.-%, insbesondere 5 bis 30 Gew.-% an optisch aktivem Monomer (I), bezogen auf das Gesamtgewicht. Sie werden intensiv mit Lösungsmitteln für das Polymere gewaschen und im Vakuum getrocknet.

Selbstverständlich können auch hier Gemische von zwei oder mehreren der erfindungsgemäßen schwefelhaltigen N-Acryloyl-aminosäurederivate, gegebenenfalls auch mit weiteren copolymerisierbaren

Monomeren eingesetzt werden.

Die Erfindung betrifft ferner die Verwendung der erfindungsgemäßen schwefelhaltigen Polyacrylamide als solche oder in vernetzter bzw. an Kieselgel gebundener Form zur chromatographischen Trennung von racemischen Gemischen in die optischen Antipoden. Besonders bewährt haben sich die erfindungsgemäßen Polymerisate zur chromatographischen Trennung von Hexahydrocarbazol-Derivaten wie z.B. 3-r-(4-Fluorphenylsulfonamido)-9-(2-carboxyethyl)-1,2,3,4,4a,9a-hexahydrocarbazol, von N-3,5-dinitrobenzoylderivatisierten Aminosäuren, Benzodiazepinen wie Oxazepam, Arylpropionsäuren und -amide, wie Ketoprofen und Ibuprofenamid, Dihydropyridinen wie z.B. 1,4-Dihydro-2,6-dimethyl-4-(2-trifluormethylphenyl)pyridin-3,5-dicarbonsäure-5-methylester und Trifluoranthrylethanol.

Die Zusammensetzung des Fließmittels kann je nach Art und Eigenschaften des zu trennenden Racemates in üblicher Weise ausgewahlt und optimiert werden Die im Kieselgel gebundenen erfindungsgemäßen schwefelhaltigen Polyacrylamide können für chromatographische Racemattrennungen unter HPLC-Bedingungen eingesetzt werden.

Die Fähigkeit der Polymerisate zur Racemattrennung wird durch die Kapazitätsverhältnisse ($k'_{1(2)}$-Werte) für die beiden Enantiomere (1) und (2) und den daraus resultierenden Enantioselektivitätswert $\alpha$ ausgedrückt. Diese chromatographischen Parameter sind wie folgt definiert:

$$\text{Kapazitätsverhältnis } k'_{1(2)} = \frac{t_{1(2)} - t_o}{t_o}$$

$$\text{Enantioselektivität } \alpha = \frac{k'_2}{k'_1}$$

$t_o$ = Totzeit der Säule

$t_{1(2)}$ = Retentionszeit des zuerst eluierten Enantiomers 1 bzw. des später eluierten Enantiomers 2

Die präparative Trennung von racemischen Gemischen in ihre optischen Antipoden unter Verwendung der erfindungsgemäßen Polymerisate wird vorzugsweise säulenchromatographisch vorgenommen. Besonders vorteilhaft ist es hierfür, die chromatographische Trennung mit Perlpolymerisaten einer bestimmten Korngrößenverteilung vorzunehmen; gute Trennleistungen werden mit Perlpolymerisaten einer Korngrößenverteilung von 5 bis 200 µm, bevorzugt 15 bis 100 µm erhalten.

Die Arbeitsmethodik der säulenchromatographischen Trennung ist bekannt, Üblicherweise wird das Polymerisat in Fließmittel suspendiert und die Suspension in eine Glassäule gefüllt. Nach Ablaufen des Fließmittels wird das zu trennende Racemat, gelöst im Fließmittel, auf die Säule aufgebracht. Dann wird mit Fließmittel eluiert und die Enantiomeren im Eluat photometrisch und/oder polarimetrisch mittels geeigneter Durchflußzellen detektiert.

Als Fließmittel werden üblicherweise organische Lösungsmittel bzw. Lösungsmittelgemische verwendet, die das als Adsorbens eingesetzte Polymerisat anquellen und das zu trennende Racemat lösen. Beispielsweise seien genannt: Kohlenwasserstoffe wie Benzol, Toluol oder Xylol, Ether wie Diethylether, tert.-Butylmethylether, Dioxan oder Tetrahydrofuran, Halogenkohlenwasserstoffe wie Di- oder Trichlormethan, Aceton, Acetonitril oder Essigester, Alkohole wie Methanol, Ethanol, n-Propanol, Isopropanol oder n-Butanol, oder aber Gemische der genannten Lösungsmittel. Als besonders geeignet haben sich Mischungen aus Toluol mit Tetrahydrofuran, Dioxan oder Isopropanol erwiesen.

Beispiele

I. Herstellungsmethode der optisch aktiven schwefelhaltigen N-Acryloylaminosäurederivate (I) (Monomere)

Beispiel 1

Zur Lösung von 23 g (80 mmol) L-Methionin-d-menthylester in 700 ml Dichlormethan werden bei 0 °C 8,5 g (84 mmol) Triethylamin gegeben. Dann wird bei -10 °C eine Lösung von 8,5 g (82 mmol) Methacryloylchlorid in 50 ml Dichlormethan zugetropft. Man läßt auf Raumtemperatur kommen und wäscht anschließend mit Wasser: 1N-Salzsäure und gesättigter Natriumhydrogencarbonatlösung. Die organische Phase wird über Magnesiumsulfat getrocknet. Nach dem Abdestillieren des Lösungsmittels wird der Rückstand aus n-Heptan umkristallisiert.

Man erhält 24 g (85 %) N-Methacryloyl-L-methionin-d-menthylester in Form farbloser Kristalle vom Fp.: 47 °C. Drehwert $[\alpha]_D$: +66,2 ° (c = 1, $CHCl_3$).

Der Rückstand kann statt durch Kristallisation auch durch Chromatographie an Kieselgel mit Hexan : Essigester = 3:1 als Eluent gereinigt werden.

Statt des freien Aminoesters kann mit gleichem Erfolg auch das entsprechende Hydrochlorid eingesetzt werden. Die Triethylaminmenge muß dann verdoppelt werden.

Beispiel 2

Zur Lösung von 21,4 g (75 mmol) L-Methionin-(1S)-bornylester in 650 ml Dichlormethan werden bei 0 °C 8,1 g (80 mmol) Triethylamin gegeben. Dann wird bei -10 °C eine Lösung von 8,7 g (80 mmol) Fluoracryloylchlorid in 50 ml Dichlormethan zugetropft. Man läßt auf Raumtemperatur kommen. Anschließend wäscht man mit Wasser, 1N-Salzsäure und gesättigter Natriumhydrogencarbonatlösung. Die organische Phase wird über Magnesiumsulfat getrocknet. Nach Abdestillieren des Lösungsmittels wird der Rückstand durch Flash-Chromatographie an Kieselgel mit Petrolether : Essigester = 3:1 als Eluent gereinigt.

Man erhält 17 g (63 %) N-Fluoracryloyl-L-methionin-(1S)-bornylester als farbloses Öl mit dem Drehwert $[\alpha]_D$ = +2,0 °C (c = 1, $CHCl_3$).

Beispiel 3

Zur Lösung von 8,1 g (30 mmol) (R)-Thioprolin-l-menthylamid in 100 ml Dichlormethan werden bei 0 °C 3,5 g (35 mmol) Triethylamin gegeben. Dann wird bei -10 °C eine Lösung von 3,1 g (34 mmol) Acrylsäurechlorid in 20 ml Dichlormethan zugetropft. Man läßt auf Raumtemperatur kommen.

Aufarbeitung wie unter Beispiel 1 beschrieben. Man erhält 6 g (64 %) N-Acryloyl-(R)-thioprolin-l-menthylamid in Form farbloser Kristalle vom Fp. 149 °C (aus n-Heptan). Drehwert $[\alpha]_D$: -184,5 ° (c = 1, $CHCl_3$).

In der genannten Art und Weise wurden aus den in Tabelle I angegebenen, optisch aktiven schwefelhaltigen Aminosäurederivaten N-(Meth)Acryloylaminosäurederivate erhalten. Die Ausbeuten, Schmelzpunkte und Drehwerte der Produkte sind ebenfalls in der Tabelle enthalten.

Tabelle I: Monomersynthese

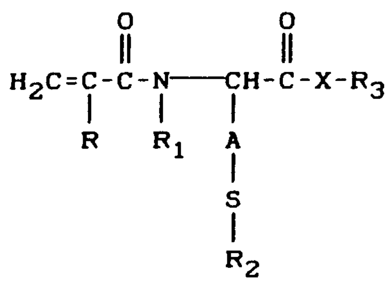

(I)

| Beispiel | Rest-Gruppen | Edukt | Produkt | Fp.(°C) | $[\alpha]_D$ ($c=1, CHCl_3$) | Ausbeute (% d.Th.) |
|---|---|---|---|---|---|---|
| 1 | $R=CH_3$, $R_1=H$, $R_2=CH_3$, $R_3$=d-Menthyl $X=O$, $A=CH_2-CH_2$ | | | | vgl. Text | |
| 2 | $R=F$, $R_1=H$ $R_2=CH_3$, $R_3$=(1S)-Bornyl $X=O$, $A=CH_2-CH_2$ | | | | dito | |
| 3 | $R=H$, $R_1-R_2=CH_2$ $R_3$= l-Menthyl, $X=NH$, $A=CH_2$ | | | | dito | |
| 4 | $R=H$, $R_1=H$ $R_2=CH_3$, $R_3$=l-Menthyl $X=O$, $A=CH_2-CH_2$ | L-Methionin-l-menthylester | N-Acryloyl-L-methionin-l-menthylester | 87 | $-19,5^0$ | 90 |

Tabelle I: Monomersynthese - Fortsetzung -

| Beispiel | Rest-Gruppen | Edukt | Produkt | Fp. ($^0$C) | $[\alpha]_D$ (c=1, $CHCl_3$) | Ausbeute (% d.Th.) |
|---|---|---|---|---|---|---|
| 5 | R=H, $R_1$-$R_2$=$CH_2$ $R_3$=d-Menthyl X=O, A=$CH_2$ | (R)-Thioprolin-d-menthylester | N-Acryloyl-(R)-thioprolin-d-menthylester | Öl | -29,5$^0$ | 72 |
| 6 | R=H, $R_1$-$R_2$=$CH_2$ $R_3$=l-Menthyl X=O, A=$CH_2$ | (R)-Thioprolin-l-menthylester | N-Acryloyl-(R)-thioprolin-l-menthylester | Öl | -115,3$^0$ | 90 |
| 7 | R=$CH_3$, $R_1$=H $R_2$=$CH_3$, $R_3$=$^t$Bu X=O, A=$CH_2CH_2$ | L-Methionin-tert.-butylester | N-Methacryloyl-L-methionin-tert.-butylester | 58 | +45,0$^0$ | 91 |
| 8 | R=$CH_3$, $R_1$=H $R_2$=$CH_3$, $R_3$=l-Menthyl, X=O, A=$CH_2CH_2$ | L-Methionin-l-menthylester | N-Methacryloyl-L-methionin-l-menthylester | 80 | -27,3$^0$ | 81 |
| 9 | R=$CH_3$, $R_1$=H $R_2$=$CH_3$, $R_3$=l-Menthyl, X=NH, A=$CH_2CH_2$ | L-Methionin-l-menthylamid | N-Methacryloyl-L-methionin-l-menthylamid | 161 | -68,3$^0$ | 65 |

<u>Tabelle I</u>: Monomersynthese - Fortsetzung -

| Beispiel | Rest-Gruppen | Edukt | Produkt | Fp. (°C) | $[\alpha]_D$ ($c=1$, $CHCl_3$) | Ausbeute (% d.Th.) |
|---|---|---|---|---|---|---|
| 10 | R=H, $R_1$=H, $R_2=CH_3$, $R_3$=l-Menthyl X=NH, $A=CH_2-CH_2$ | L-Methionin-l-menthylamid | N-Acryloyl-L-methionin-l-menthylamid | 167 | $-76,1^0$ | 63 |
| 11 | $R=CH_3$, $R_1$=H $R_2=CH_3$, $R_3$=d-Menthyl X=NH, $A=CH_2-CH_2$ | L-Methionin-d-menthylamid | N-Methacryloyl-L-methionin-d-menthylamid | 192 | $+38,4^0$ | 85 |
| 12 | R=H, $R_1$=H $R_2=CH_3$, $R_3$=d-Menthyl, X=NH, $A=CH_2CH_2$ | L-Methionin-d-menthylamid | N-Acryloyl-L-methionin-d-menthylamid | 209 | $+19,1^0$ | 55 |
| 13 | $R=CH_3$, $R_1-R_2=CH_2$ $R_3$=l-Menthyl, X=NH, $A=CH_2$ | (R)-Thioprolin-l-menthylamid | N-Methacryloyl-(R)-thioprolin-l-menthylamid | 154 | $-195,3^0$ | 65 |
| 14 | R=H, $R_1$=H $R_2=CH_3$, $R_3$=d-Menthyl, X=O, $A=CH_2-CH_2$ | L-Methionin-d-menthylester | N-Acryloyl-L-methionin-d-menthylester | 74 | $+83,6^0$ | 67 |
| 15 | R=H, $R_1-R_2=CH_2$ $R_3$=d-Menthyl, X=NH, $A=CH_2$ | (R)-Thioprolin-d-menthylamid | N-Acryloyl-(R)-thioprolin-d-menthylamid | 110 | $-78,8^0$ | 59 |

<u>Tabelle I</u>: Monomersynthese - Fortsetzung -

| Beispiel | Rest-Gruppen | Edukt | Produkt | Fp. ($^0$C) | $[\alpha]_D$ (c=1, $CHCl_3$) | Ausbeute (% d.Th.) |
|---|---|---|---|---|---|---|
| 16 | $R=CH_3$, $R_1=H$ $R_2=CH_2CH_3$, $R_3$=d-Menthyl, X=NH, $A=CH_2CH_2$ | D-Ethionin-d-menthylamid | N-Methacryloyl-D-ethionin-d-menthylamid | 127 | -73,7$^0$ | 66 |
| 17 | $R=CH_3$, $R_1=H$ $R_2=CH_2CH_3$, $R_3$=l-Menthyl, X=NH, $A=CH_2CH_2$ | D-Ethionin-l-menthylamid | N-Methacryloyl-D-ethionin-l-menthylamid | 178 | -28,3$^0$ | 44 |
| 18 | $R=CH_3$, $R_1=H$, $R_2=CH_3$, $R_3$=(S)-Phenylethyl X=NH, $A=CH_2CH_2$ | L-Methionin-(S)-phenylethylamid | N-Methacryloyl-L-methionin-(S)-phenylethylamid | 137 | -52,6$^0$ | 77 |
| 19 | $R=CH_3$, $R_1=H$, $R_2=CH_3$, $R_3$=d-Neomenthyl X=NH, $A=CH_2CH_2$ | L-Methionin-d-neomenthylamid | N-Methacryloyl-L-methionin-d-neomenthylamid | 170 | +9,1$^0$ | 83 |
| 20 | $R=CH_3$, $R_1=H$, $R_2=CH_3$, $R_3$=(R)-Phenylethyl X=NH, $A=CH_2CH_2$ | L-Methionin-(R)-phenylethylamid | N-Methacryloyl-L-methionin-(R)-phenylethylamid | 121 | +7,8$^0$ | 56 |

Tabelle I: Monomersynthese - Fortsetzung -

| Beispiel | Rest-Gruppen | Edukt | Produkt | Fp. (°C) | $[\alpha]_D$ (c=1, $CHCl_3$) | Ausbeute (% d.Th.) |
|---|---|---|---|---|---|---|
| 21 | $R=CH_3$, $R_1=H$, $R_2=CH_3$, $R_3$=Phenyl, X=NH, $A=CH_2CH_2$ | L-Methionin-anilid | N-Meth-acryloyl-L-methionin-anilid | 117 | -29,8° | 88 |
| 22 | $R=CH_3$, $R_1=H$, $R_2=CH_3$, $R_3$=3,5-Dimethylphenyl, X=NH, $A=CH_2CH_2$ | L-Methionin-3,5-dimethyl-anilid | N-Meth-acryloyl-L-methionin-3,5-dimethyl-anilid | 114 | -18,1° | 80 |
| 23 | $R=CH_3$, $R_1=H$, $R_2=CH_3$, $R_3$=3-Pentyl X=NH, $A=CH_2CH_2$ | L-Methionin-3-pentylamid | N-Meth-acryloyl-L-methionin-3-pentylamid | 128 | -21,8° | 66 |
| 24 | $R=CH_3$, $R_1=H$, $R_2=CH_3$, $R_3$=1-Menthyl X=NH, $A=CH_2$ | S-Methyl-L-cystein-1-menthylamid | N-Meth-acryloyl-S-methyl-L-cystein-1-menthylamid | 155 | -81,4° (MeOH) | 63 |
| 25 | R=H, $R_1$-$R_2$=$CH_2$ $R_3$=(S)-Phenylethyl X=NH, $A=CH_2$ | (R)-Thioprolin-(S)-phenylethylamid | N-Acryloyl-(R)-thioprolin-(S)-phenylethylamid | 112 | -164,7° | 30 |

II. Polymerisation der optisch aktiven schwefelhaltigen Aminosäurederivate (I)

1. Herstellung in an Kieselgel gebundener Form

3,0 g Vinylkieselgel (hergestellt aus Kieselgel Si 100/5 μ bzw. 10 μ nach H.Engelhardt et al., Chromatographia 27 (1989) 535) werden mit 6,0 g schwefelhaltigem Aminosäurederivat I und 100 mg Azobisisobuttersäurenitril in 25 ml Toluol gelöst bzw. suspendiert. Unter Magnetrührung wird die Apparatur dreimal evakuiert und mit Stickstoff gefüllt. Die Mischung wird dann 45 Minuten bei 80 °C polymerisiert. Nach Zusatz von 100 mg 2,6-Di-tert.-butyl-4-methylphenol wird rasch auf Raumtemperatur abgekühlt. Das Kieselgel wird über eine Fritte G4 abgesaugt, zweimal in Chloroform, einmal in Toluol und einmal in Isopropanol je 15 Minuten verrührt und zwischendurch abgesaugt. Das Kieselgel wird anschließend bei Raumtemperatur im Vakuum (<0,005 at) getrocknet.

In der nachstehenden Tabelle II sind die aufpolymerisierten schwefelhaltigen Aminosäurederivate, die Ausbeuten an optisch aktivem Kieselgel, dessen Stickstoffgehalt und daraus folgend der Gehalt an gebundenem Polymer zusammengestellt.

Ähnliche Ergebnisse an gebundenem optisch aktivem Polymer erhält man bei Verwendung von mit Methacrylsäureanhydrid verestertem Kieselgeldiol anstelle von Vinylkieselgel.

Tabelle II: Kieselgelphasen

| Beispiel | Monomer gemäß Beispiel-Nr. | Ausbeute (g) | N-Gehalt (%) | Gehalt an gebundenem Polymerisat (Gew.-%) |
|---|---|---|---|---|
| 1A | 1 | 3,5 | 0,6 | 15,2 |
| 2A | 2 | 3,5 | 0,7 | 17,8 |
| 3A | 3 | 3,45 | 1,5 | 17,4 |
| 4A | 4 | 3,2 | 0,55 | 13,4 |
| 6A | 6 | 3,3 | 0,65 | 15,1 |
| 7A | 7 | 3,4 | 0,8 | 15,6 |
| 8A | 8 | 3,4 | 0,6 | 15,2 |
| 9A | 9 | 3,2 | 0,8 | 10,1 |
| 10A | 10 | 3,3 | 1,35 | 16,4 |
| 11A | 11 | 3,4 | 1,2 | 15,2 |
| 12A | 12 | 3,5 | 1,4 | 17,0 |
| 13A | 13 | 3,1 | 0,2 | 2,4 |
| 14A | 14 | 3,2 | 0,65 | 15,9 |
| 15A | 15 | 3,6 | 2,0 | 23,2 |
| 16A | 16 | 3,3 | 0,5 | 6,6 |
| 17A | 17 | 3,4 | 1,2 | 15,8 |
| 18A | 18 | 3,5 | 1,8 | 20,6 |
| 19A | 19 | 3,3 | 1,15 | 14,5 |
| 20A | 20 | 3,4 | 1,5 | 17,2 |
| 21A | 21 | 3,4 | 1,45 | 15,2 |
| 22A | 22 | 3,5 | 1,5 | 17,1 |
| 23A | 23 | 3,4 | 1,75 | 17,9 |
| 24A | 24 | 3,1 | 1,3 | 15,8 |

2. Herstellung in Form von Perlpolymerisaten

Die Lösung von 13,5 g schwefelhaltigem Aminosäurederivat I, 1,5 g Ethylenglykoldimethacrylat und 0,3 g Azobisisobuttersäurenitril in 45 g Chloroform wird unter Rühren mit 450 U/min in einer Lösung von 5 g Polyvinylalkohol in 130 ml Wasser dispergiert. Die Apparatur wird mehrmals evakuiert und mit Stickstoff gefüllt. Anschließend wird 16 h bei 55 °C unter Stickstoff polymerisiert. Das Polymerisationsgemisch wird dann in 2 bis 3 l Wasser eingerührt und die flüssige Phase nach dem Absitzen des Perlpolymerisats dekantiert. Das Perlpolymerisat wird durch 3 bis 4-maliges Suspendieren in Wasser und Abdekantieren der flüssigen Phase vom Feinkorn (Polymerisat mit einer Korngröße von <10 µm) befreit und nach intensivem Waschen mit Aceton bei 60 °C bis zur Gewichtskonstanz getrocknet.

In der nachstehenden Tabelle III sind die für die Polymerisation verwendeten schwefelhaltigen Aminosäurederivate, die Ausbeuten, in denen die Polymerisate erhalten wurden, deren Korngröße und das Volumen der erhaltenen Perlpolymerisate in trockenem ($V_s$) und gequollenem ($V_q$) Zustand (Quellmittel: Toluol = T bzw. Toluol/THF = 3:2 V/V-Gemisch = T/T) zusammengestellt.

Tabelle III: Perlpolymerisate

| Beispiel | Monomer gemäß Beispiel Nr. | Ausbeute (g) | Korngröße (µm) | $V_s$ (ml/g) | $V_q$ (ml/g) |
|---|---|---|---|---|---|
| 8B | 8 | 12,0 | 15-60 | 1,9 | 5,1 |
| 9B | 9 | 12,1 | 10-50 | 1,7 | 5,9 |
| 10B | 10 | 11,4 | 10-30 | 1,9 | 6,7 |
| 11B | 11 | 11,7 | 15-60 | 2,0 | 5,7 |
| 18B | 18 | 13,6 | 10-60 | 1,8 | 4,4 |
| 19B | 19 | 11,5 | 20-90 | 1,9 | 4,9 |
| 20B | 20 | 11,7 | 15-85 | 1,8 | 4,6 |
| 23B | 23 | 11,0 | 15-85 | 1,9 | 4,4 |

III. Verwendung der optisch aktiven Polymerisate der schwefelhaltigen Aminosäurederivate (I) als Adsorbentien für die Racemattrennung

Für die chromatographischen Trennungen wurden folgende Testracemate verwendet:

Racemat Nr. 1: 3-r-(4-Fluorphenylsulfonamido)-9-(2-carboxyethyl)-1,2,3,4,4a,9a-hexahydrocarbazol
Racemat Nr. 2: Oxazepam
Racemat Nr. 3: Ketoprofen
Racemat Nr. 4: 1,4-Dihydro-2,6-dimethyl-4-(2-trifluormethylphenyl)pyridin-3,5-dicarbonsäure-5-methylester
Racemat Nr. 5: N-3,5-Dinitrobenzoylleucin
Racemat Nr. 6: Trifluoranthrylethanol
Racemat Nr. 7: Ibuprofenamid

Die an Kieselgel gebundenen Polymerisate wurden in Stahlsäulen (Innendurchmesser: 4 mm; Länge: 25 cm) eingesetzt. Eluiert wurde mit n-Heptan-Tetrahydrofuran-Gemischen: 1:1 (vol/vol) = Elutionsmittel a, 1:2 = Elutionsmittel b. Die Fließmittelgeschwindigkeit betrug 1 ml/min.

Die Perlpolymerisate wurden in einer Glassäule (Innendurchmesser: 1,2 cm; Betthöhe: 25-30 cm) eingesetzt. Eluiert wurde mit einem Toluol-tetrahydrofuran-Gemisch 3:1 (vol/vol). Die Fließmittelgeschwindigkeit betrug 0,5 ml/min.

Die bei der chromatographischen Trennung der verschiedenen Testracemate erhaltenen Ergebnisse (Enantioselektivität $\alpha$ und Kapazitätsverhältnis $k'_1$) und die verwendeten Elutionsmittel sind in Tabelle IV zusammengestellt. Dabei wird ein Beispiel für die erfindungsgemäßen Adsorbentien im Vergleich mit der analogen schwefelfreien Phase (siehe EP-A 379 917) gegeben. Es zeigt sich, daß die schwefelfreie Norleucinphase der erfindungsgemäßen schwefelhaltigen Methioninphase unterlegen ist.

Tabelle IV: Trennergebnisse

Stationäre Phase

| | aus N-Methacryloyl-L-methionin-1-menthylamid (→ 9A) | | | aus N-Methacryloyl-L-norleucin 1-menthylamid (EP 379 917) | | |
|---|---|---|---|---|---|---|
| | 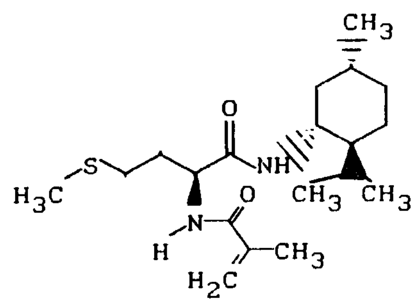 | | | 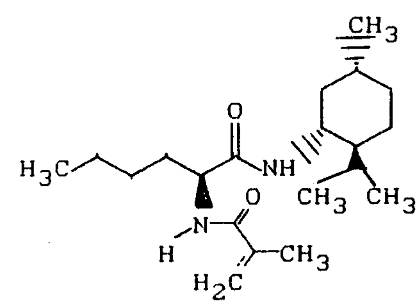 | | |
| Racemat | Eluens | $k'_1$ | alpha | Eluens | k' | alpha |
| Nr. 1 | Heptan:THF = 1:2 | 2,88 | 5,00 | keine Trennung | | |
| Nr. 2 | dito = 1:1 | 2,67 | 4,32 | Heptan:THF = 1:2 | 4,00 | 1,34 |
| Nr. 3 | dito = 3:1 | 3,06 | 1,09 | keine Trennung | | |
| Nr. 4 | dito = 1:1 | 1,64 | 2,14 | dito = 1:1 | 2,77 | 1,68 |
| Nr. 5 | dito = 1:1 | 0,56 | 1,63 | dito = 1:1 | 0,63 | 1,17 |
| Nr. 6 | Heptan:Isopropanol = 10:1 | 1,33 | 1,26 | Heptan:Isopropanol = 10:1 | 1,13 | 1,16 |
| Nr. 7 | Heptan:THF = 1:1 | 1,74 | 1,82 | Heptan:THF = 1:1 | 1,99 | 1,77 |

**Patentansprüche**

**Patentansprüche für folgende Vertragsstaaten : AT, BE, CH, DE, DK, FR, GB, IT, LI, LU, NL, PT, SE**

1. Optisch aktive schwefelhaltige Aminosäurederivate der Formel (I)

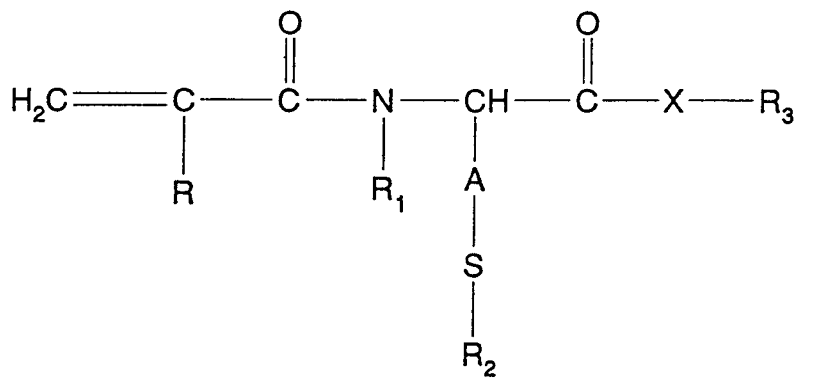

(I)

in welcher

R für Wasserstoff, Methyl oder Fluor steht,

$R_1$ Wasserstoff oder Methyl bedeutet oder zusammen mit $R_2$ eine Methylengruppe, die ein- oder zweifach Methyl- oder einfach tertiär -Butyl-substituiert sein kann, oder eine Dimethylengruppe bildet,

$R_2$ für Alkyl mit bis zu 8 C-Atomen, Phenyl,

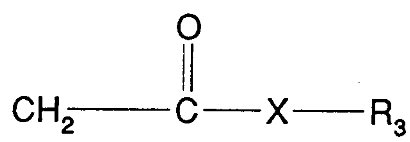

steht oder zusammen mit $R_1$ eine dort beschriebene Brücke bildet,

$R_3$ für einen $C_{10}$-Terpenylrest, einen Adamantylrest oder einen Decahydronaphthylrest steht oder für einen Alkylrest oder Cycloalkylrest mit jeweils bis zu 12 C Atomen mit der Ausnahme von Methyl und Ethyl steht, der gegebenenfalls ein- oder zweifach substituiert ist durch $C_6$-$C_{10}$-Aryl, $C_1$-$C_4$-Alkoxy, $C_3$-$C_{12}$-Cycloalkyl oder Halogen, wobei die genannten Aryl- und Cycloalkylreste ihrerseits wieder durch $C_1$-$C_4$-Alkyl substituiert sein können,

X Sauerstoff oder eine $NR_4$-Gruppe bedeutet, in der $R_4$ für Wasserstoff oder $C_1$-$C_4$-Alkyl steht oder zusammen mit $R_3$ einen stickstoffhaltigen 5- bis 7-gliedrigen Ring bildet, der gegebenenfalls ein- oder zweifach $C_1$-$C_4$-alkyl- oder $C_1$-$C_6$-alkoxycarbonylsubstituiert sein kann,

A für eine Methylen- oder eine Dimethylengruppe steht.

2. Optisch aktive schwefelhaltige Aminosäurederivate aus der Gruppe der Beispiele 1 bis 25 gemäß Tabelle I in der Beschreibung.

3. Verbindungen der allgemeinen Formel (I) gemäß Anspruch 1, dadurch gekennzeichnet, daß sie die Aminosäuresequenz der optisch aktiven schwefelhaltigen Aminosäuren Cystein, Homocystein, Penicillamin enthalten und die SH-Funktion alkyliert, aryliert, alkoxycarbonylmethyliert oder über eine Alkylenbrücke mit der Aminogruppe verbunden ist.

**4.** Verfahren zur Herstellung von Verbindungen der allgemeinen Formel (I)

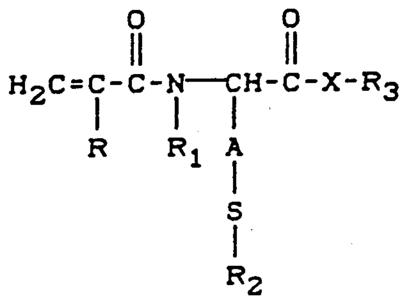 (I),

in der R, $R_1$, $R_2$, $R_3$, A und X die in Anspruch 1 angegebene Bedeutung haben, dadurch gekennzeichnet, daß man optisch aktive schwefelhaltige Aminosäurederivate der Formel (II)

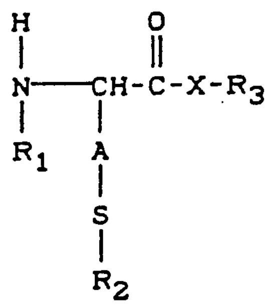 (II)

in der $R_1$, $R_2$, $R_3$, A und X die unter Formel (I) angegebene Bedeutung haben, oder deren Säureadditionsprodukte mit Acryloyl-Derivaten der Formel (III)

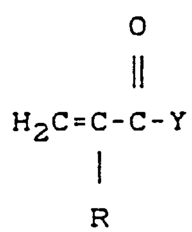 (III),

in der R die unter Formel (I) angegebene Bedeutung hat und

Y für Fluor, Chlor oder Brom steht oder für den Rest

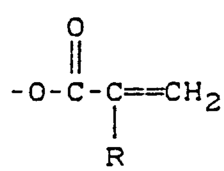

steht (Anhydrid),

gegebenenfalls in Gegenwart eines Säurebindemittels in inerten organischen Lösungsmitteln umsetzt.

**5.** Optisch aktive Polymere, enthaltend mindestens 40 Mol-% Struktureinheiten der Formel (IV)

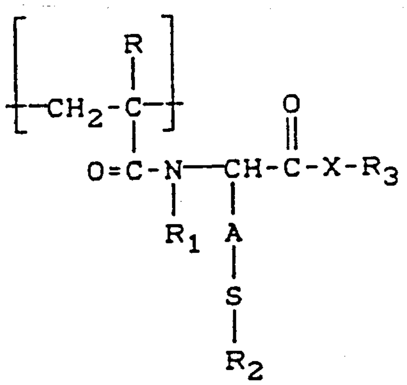

(IV)

in welcher

| | |
|---|---|
| R | für Methyl oder Fluor steht, und |
| $R_1$, $R_2$, $R_3$, A und X | die in Anspruch 1 angegebene Bedeutung haben. |

**6.** Optisch aktive Polymere gemäß Anspruch 5, dadurch gekennzeichnet, daß sie in Form von vernetzten unlöslichen aber quellbaren Perlpolymerisaten oder in an feinteilige anorganische Trägermaterialien gebundener Form vorliegen.

**7.** Verfahren zur Herstellung von vernetzten Perlpolymerisaten gemäß Anspruch 6, dadurch gekennzeichnet, daß man die schwefelhaltigen N-Acryloyl-aminosäure-Derivate der allgemeinen Formel (I) mit 0,5 bis 50 Mol-% eines Vernetzers durch Suspensionspolymerisation, gegebenenfalls in Anwesenheit von Radikalbildnern und inerten organischen Lösungsmitteln herstellt.

**8.** Verfahren zur Herstellung von an anorganische Trägermaterialien gebundenen optisch aktiven Polymeren gemäß Anspruch 6, dadurch gekennzeichnet, daß man Verbindungen der allgemeinen Formel (I) gemäß Anspruch 1 in Gegenwart von gegebenenfalls (Meth)acryloyl-substituierten Kieselgel-Diolphasen oder von Kieselgel-Vinylphasen gegebenenfalls in Anwesenheit von inerten organischen Lösungsmitteln polymerisiert.

**9.** Verwendung von optisch aktiven Polymeren, enthaltend mindestens 40 Mol-% Struktureinheiten der Formel IV gemäß Anspruch 6, in welcher

| | |
|---|---|
| R | für Wasserstoff, Methyl oder Fluor steht, |
| $R_1$, $R_2$, A und X | die in Anspruch 1 angegebene Bedeutung haben, und |
| $R_3$ | für einen $C_{10}$-Terpenylrest, einen Adamantylrest oder einen Decahydronaphthylrest steht oder für einen Alkylrest oder Cycloalkylrest mit jeweils bis zu 12 C Atomen steht, der gegebenenfalls ein- oder zweifach substituiert ist durch $C_6$-$C_{10}$-Aryl, $C_1$-$C_4$-Alkoxy, $C_3$-$C_{12}$-Cycloalkyl oder Halogen, wobei die genannten Aryl- und Cycloalkylreste ihrerseits wieder durch $C_1$-$C_4$-Alkyl substituiert sein können, |

zur Trennung von racemischen Gemischen in die optischen Antipoden.

**Patentansprüche für folgende Vertragsstaaten : ES, GR**

**1.** Verfahren zur Herstellung von Verbindungen der allgemeinen Formel (I)

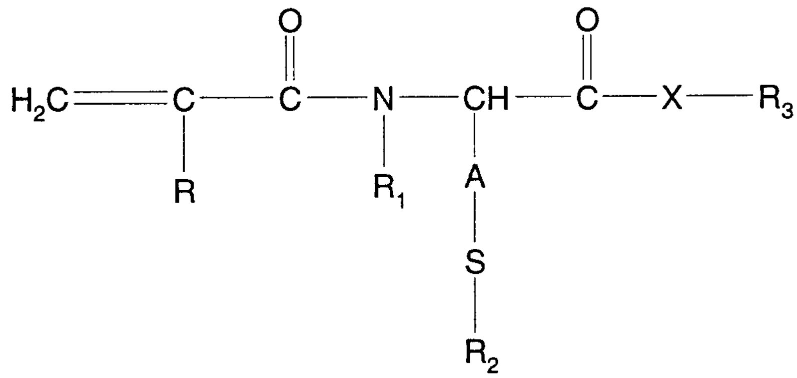

(I)

in welcher

R für Wasserstoff, Methyl oder Fluor steht,

$R_1$ Wasserstoff oder Methyl bedeutet oder zusammen mit $R_2$ eine Methylengruppe, die ein- oder zweifach Methyl- oder einfach tertiär -Butyl-substituiert sein kann, oder eine Dimethylengruppe bildet,

$R_2$ für Alkyl mit bis zu 8 C-Atomen, Phenyl,

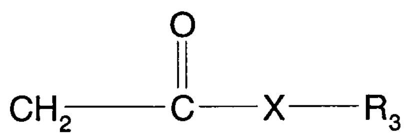

steht oder zusammen mit $R_1$ eine dort beschriebene Brücke bildet,

$R_3$ für einen $C_{10}$-Terpenylrest, einen Adamantylrest oder einen Decahydronaphthylrest steht oder für einen Alkylrest oder Cycloalkylrest mit jeweils bis zu 12 C Atomen mit der Ausnahme von Methyl und Ethyl steht, der gegebenenfalls ein- oder zweifach substituiert ist durch $C_6$-$C_{10}$-Aryl, $C_1$-$C_4$-Alkoxy, $C_3$-$C_{12}$-Cycloalkyl oder Halogen, wobei die genannten Aryl- und Cycloalkylreste ihrerseits wieder durch $C_1$-$C_4$-Alkyl substituiert sein können,

X Sauerstoff oder eine $NR_4$-Gruppe bedeutet, in der $R_4$ für Wasserstoff oder $C_1$-$C_4$-Alkyl steht oder zusammen mit $R_3$ einen stickstoffhaltigen 5- bis 7-gliedrigen Ring bildet, der gegebenenfalls ein- oder zweifach $C_1$-$C_4$-alkyl- oder $C_1$-$C_6$-alkoxycarbonylsubstituiert sein kann,

A für eine Methylen- oder eine Dimethylengruppe steht,

dadurch gekennzeichnet, daß man optisch aktive schwefelhaltige Aminosäurederivate der Formel (II)

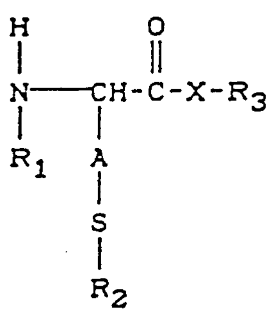

(II)

in der $R_1$, $R_2$, $R_3$, A und X die unter Formel (I) angegebene Bedeutung haben, oder deren Säureadditionsprodukte mit Acryloyl-Derivaten der Formel (III)

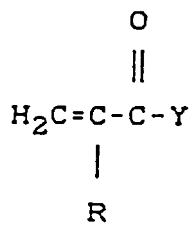 (III),

in der R die unter Formel (I) angegebene Bedeutung hat und

Y für Fluor, Chlor oder Brom steht oder für den Rest

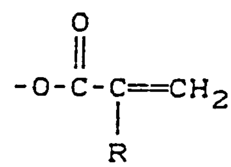

steht (Anhydrid),

gegebenenfalls in Gegenwart eines Säurebindemittels in inerten organischen Lösungsmitteln umsetzt.

**2.** Verfahren zur Herstellung von optisch aktiven Polymeren, enthaltend mindestens 40 Mol-% Struktureinheiten der Formel IV

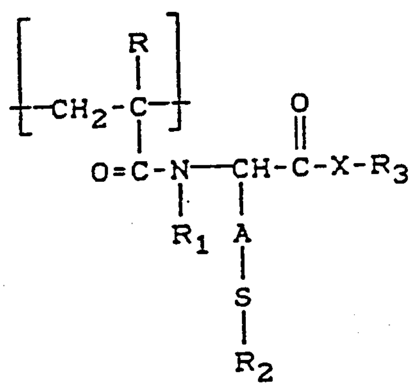 (IV)

in welcher

R für Methyl oder Fluor steht,

$R_1$, $R_2$, $R_3$, A und X die in Anspruch 1 angegebene Bedeutung haben, dadurch gekennzeichnet, daß man Verbindungen der Formel (I), gemäß Anspruch 1 nach üblichen Methoden durch Polymerisation oder Copolymerisation der Verbindungen der allgemeinen Formel (I) gemäß Anspruch 1 in vernetzte, unlösliche aber quellbare Perlpolymerisate oder in Polymere, die in gebundener Form an feinteiligen anorganischen Trägermaterialien vorliegen, überführt.

**3.** Verfahren zur Herstellung von vernetzten Perlpolymerisaten gemäß Anspruch 2, dadurch gekennzeichnet, daß man die schwefelhaltigen N-Acryloyl-aminosäure-Derivate der allgemeinen Formel (I) mit 0,5 bis 50 Mol-% eines Vernetzers durch Suspensionspolymerisation, gegebenenfalls in Anwesenheit von Radikalbildnern und inerten organischen Lösungsmitteln herstellt.

**4.** Verfahren zur Herstellung von an anorganischen Trägermaterialien gebundenen optisch aktiven Polymeren gemäß Anspruch 2, dadurch gekennzeichnet, daß man Verbindungen der allgemeinen Formel (I) gemäß Anspruch 1 in Gegenwart von gegebenenfalls (Meth)acryloyl-substituierten Kieselgel-Diolphasen oder von Kieselgel-Vinylphasen gegebenenfalls in Anwesenheit von inerten organischen Lösungsmitteln polymerisiert.

5. Verwendung von optisch aktiven Polymeren, enthaltend mindestens 40 Mol-% Struktureinheiten der Formel IV gemäß Anspruch 2, in welcher

R für Wasserstoff, Methyl oder Fluor steht,

$R_1$, $R_2$, A und X die in Anspruch 1 angegebene Bedeutung haben, und

$R_3$ für einen $C_{10}$-Terpenylrest, einen Adamantylrest oder einen Decahydronaphthylrest steht oder für einen Alkylrest oder Cycloalkylrest mit jeweils bis zu 12 C Atomen steht, der gegebenenfalls ein- oder zweifach substituiert ist durch $C_6$-$C_{10}$-Aryl, $C_1$-$C_4$-Alkoxy, $C_3$-$C_{12}$-Cycloalkyl oder Halogen, wobei die genannten Aryl- und Cycloalkylreste ihrerseits wieder durch $C_1$-$C_4$-Alkyl substituiert sein können,

zur Trennung von racemischen Gemischen in die optischen Antipoden.

**Claims**

**Claims for the following Contracting States : AT, BE, CH, DE, DK, FR, GB, IT, LI, LU, NL, PT, SE**

1. Optically active sulphur-containing amino acid derivatives of the formula (I)

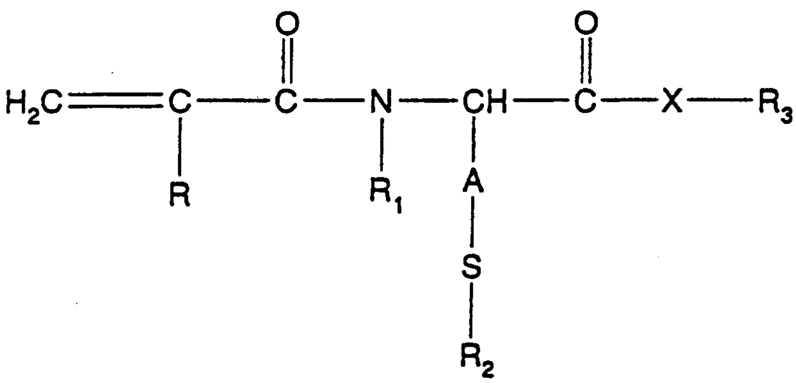

(I)

where

R represents hydrogen, methyl or fluorine,

$R_1$ is hydrogen or methyl or together with $R_2$ forms a methylene group which can be monosubstituted or disubstituted by methyl or monosubstituted by tertiary-butyl, or forms a dimethylene group,

$R_2$ represents alkyl having up to 8 carbon atoms, phenyl,

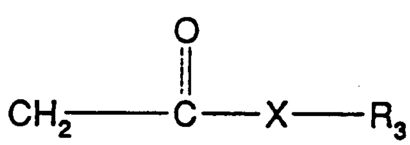

or together with $R_1$ forms a bridge described under $R_1$,

$R_3$ represents a $C_{10}$-terpenyl radical, an adamantyl radical or a decahydronaphthyl radical or represents an alkyl radical or cycloalkyl radical having, in each case, up to 12 carbon atoms with the exception of methyl and ethyl, which is optionally monosubstituted or disubstituted by $C_6$-$C_{10}$-aryl, $C_1$-$C_4$-alkoxy, $C_3$-$C_{12}$-cycloalkyl or halogen, with the specified aryl and cycloalkyl radicals in turn being able to be substituted by $C_1$-$C_4$-alkyl,

X is oxygen or an $NR_4$ group in which $R_4$ represents hydrogen or $C_1$-$C_4$-alkyl or together with $R_3$ forms a nitrogen-containing 5-membered to 7-membered ring which can optionally be monosubstituted or disubstituted by $C_1$-$C_4$-alkylcarbonyl or $C_1$-$C_6$-alkoxycarbonyl,

A represents a methylene or a dimethylene group.

2. Optically active sulphur-containing amino acid derivatives selected from those of Examples 1 to 25 as shown in Table I in the description.

3. Compounds of the general formula (I) according to Claim 1, characterized in that they contain the amino acid sequence of the optically active sulphur-containing amino acids cysteine, homocysteine, penicillamine and the SH function is alkylated, arylated, alkoxycarbonylmethylated or bonded to the amino group via an alkylene bridge.

4. Process for preparing compounds of the general formula (I)

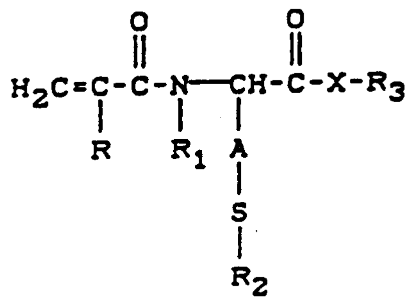

(I),

where R, $R_1$, $R_2$, $R_3$, A and X are as defined in Claim 1,
characterized in that optically active sulphur-containing amino acid derivatives of the formula (II)

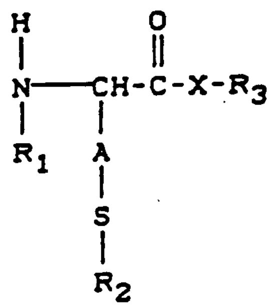

(II)

where $R_1$, $R_2$, $R_3$, A and X are as defined under formula (I),
or their acid addition products with acryloyl derivatives of the formula (III)

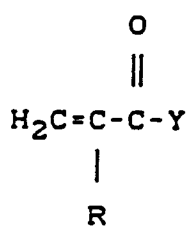

(III),

where R is as defined under formula (I) and
Y represents fluorine, chlorine or bromine or the radical

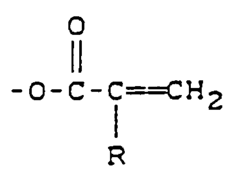

(anhydride),
are reacted, optionally in the presence of an acid binder in inert organic solvents.

**5.** Optically active polymers containing at least 40 mol% of structural units of the formula (IV)

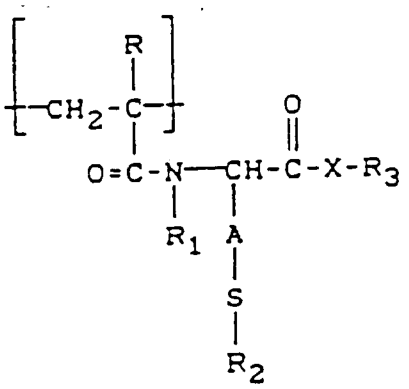

(IV)

where

| | |
|---|---|
| R | represents methyl or fluorine, and |
| $R_1$, $R_2$, $R_3$, A and X | are as defined in Claim 1. |

**6.** Optically active polymers according to Claim 5, characterized in that they are in the form of crosslinked insoluble but swellable bead polymers or in a form bound to finely divided inorganic support materials.

**7.** Process for preparing crosslinked bead polymers according to Claim 2, characterized in that the sulphur-containing N-acryloyl amino acid derivatives of the general formula (I) are prepared by suspension polymerization using 0.5 to 50 mol% of a crosslinker, optionally in the presence of free-radical formers and inert organic solvents.

**8.** Process for preparing optically active polymers bound to inorganic support materials, according to Claim 6, characterized in that compounds of the general formula (I) according to Claim 1 are polymerized in the presence of optionally (meth)acryloyl-substituted silica gel-diol phases or of silica gel-vinyl phases, optionally in the presence of inert organic solvents.

**9.** The use of optically active polymers containing at least 40 mol% of structural units of the formula IV according to Claim 6, where

| | |
|---|---|
| R | represents hydrogen, methyl or fluorine, |
| $R_1$, $R_2$, A and X | are as defined in Claim 1, and |
| $R_3$ | represents a $C_{10}$-terpenyl radical, an adamantyl radical or a decahydronaphthyl radical or represents an alkyl radical or cycloalkyl radical having, in each case, up to 12 carbon atoms, which is optionally monosubstituted or disubstituted by $C_6$-$C_{10}$-aryl, $C_1$-$C_4$-alkoxy, $C_3$-$C_{12}$-cycloalkyl or halogen, with the specified aryl and cycloalkyl radicals in turn being able to be substituted by $C_1$-$C_4$-alkyl, |

for the separation of racemic mixtures into their enantiomers.

**Claims for the following Contracting States : ES, GR**

**1.** Process for preparing compounds of the general formula (I)

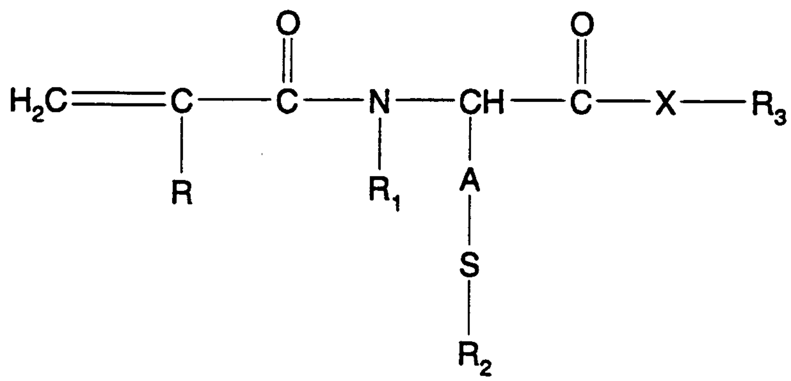

(I)

where

- R represents hydrogen, methyl or fluorine,
- $R_1$ is hydrogen or methyl or together with $R_2$ forms a methylene group which can be monosubstituted or disubstituted by methyl or monosubstituted by tertiary-butyl, or forms a dimethylene group,
- $R_2$ represents alkyl having up to 8 carbon atoms, phenyl,

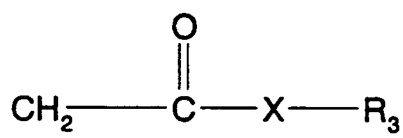

or together with $R_1$ forms a bridge described under $R_1$,
- $R_3$ represents a $C_{10}$-terpenyl radical, an adamantyl radical or a decahydronaphthyl radical or represents an alkyl radical or cycloalkyl radical having, in each case, up to 12 carbon atoms with the exception of methyl and ethyl, which is optionally monosubstituted or disubstituted by $C_6$-$C_{10}$-aryl, $C_1$-$C_4$-alkoxy, $C_3$-$C_{12}$-cycloalkyl or halogen, with the specified aryl and cycloalkyl radicals in turn being able to be substituted by $C_1$-$C_4$-alkyl,
- X is oxygen or an $NR_4$ group in which $R_4$ represents hydrogen or $C_1$-$C_4$-alkyl or together with $R_3$ forms a nitrogen-containing 5-membered to 7-membered ring which can optionally be monosubstituted or disubstituted by $C_1$-$C_4$-alkylcarbonyl or $C_1$-$C_6$-alkoxycarbonyl,
- A represents a methylene or a dimethylene group,

characterized in that optically active sulphur-containing amino acid derivatives of the formula (II)

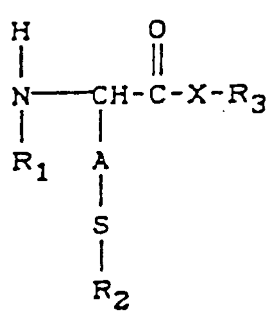

(II)

where $R_1$, $R_2$, $R_3$, A and X are as defined under formula (I),
or their acid addition products with acryloyl derivatives of the formula (III)

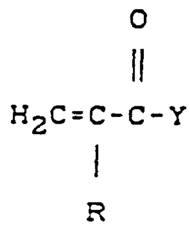 (III),

where R is as defined under formula (I) and

Y represents fluorine, chlorine or bromine or the radical

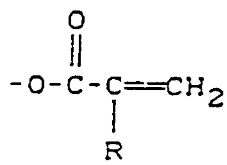

(anhydride),

are reacted, optionally in the presence of an acid binder in inert organic solvents.

**2.** Process for preparing optically active polymers containing at least 40 mol% of structural units of the formula IV

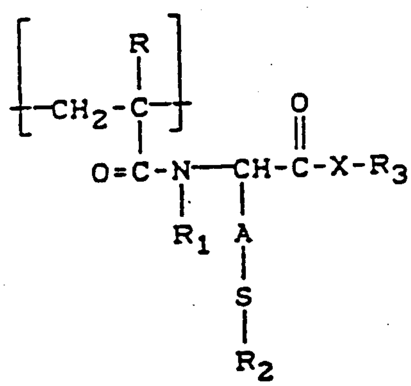 (IV)

where

| | |
|---|---|
| R | represents methyl or fluorine, |
| $R_1$, $R_2$, $R_3$, A and X | are as defined in Claim 1, characterized in that compounds of the formula (I) according to Claim 1 are converted by conventional methods by polymerization or copolymerization of the compounds of the general formula (I) according to Claim 1 into cross-linked, insoluble but swellable bead polymers or into polymers which are in a form bound to finely divided inorganic support materials. |

**3.** Process for preparing crosslinked bead polymers according to Claim 2, characterized in that the sulphur-containing N-acryloyl amino acid derivatives of the general formula (I) are prepared by suspension polymerization using 0.5 to 50 mol% of a crosslinker, optionally in the presence of free-radical formers and inert organic solvents.

**4.** Process for preparing optically active polymers bound to inorganic support materials, according to Claim 2, characterized in that compounds of the general formula (I) according to Claim 1 are polymerized in the presence of optionally (meth)acryloyl-substituted silica gel-diol phases or of silica gel-vinyl phases, optionally in the presence of inert organic solvents.

**5.** The use of optically active polymers containing at least 40 mol% of structural units of the formula IV according to Claim 2, where

R represents hydrogen, methyl or fluorine,

$R_1$, $R_2$, A and X are as defined in Claim 1, and

$R_3$ represents a $C_{10}$-terpenyl radical, an adamantyl radical or a decahydronaphthyl radical or represents an alkyl radical or cycloalkyl radical having, in each case, up to 12 carbon atoms, which is optionally monosubstituted or disubstituted by $C_6$-$C_{10}$-aryl, $C_1$-$C_4$-alkoxy, $C_3$-$C_{12}$-cycloalkyl or halogen, with the specified aryl and cycloalkyl radicals in turn being able to be substituted by $C_1$-$C_4$-alkyl,

for the separation of racemic mixtures into their enantiomers.

**Revendications**

**Revendications pour les Etats contractants suivants : AT, BE, CH, DE, DK, FR, GB, IT, LI, LU, NL, PT, SE**

**1.** Dérivés d'aminoacides optiquement actifs contenant du soufre, de formule (I)

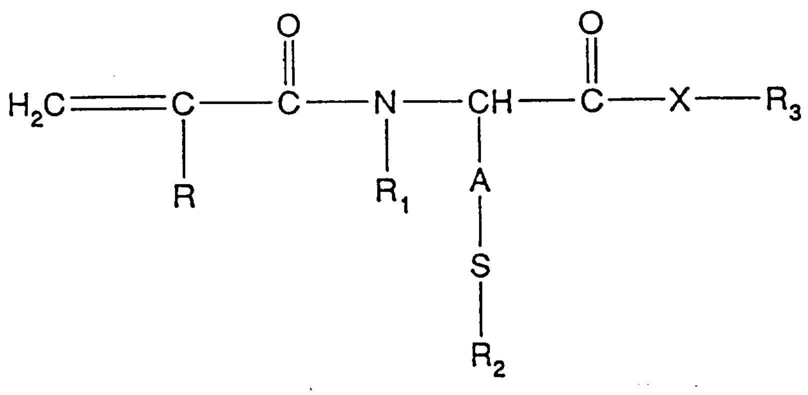

(I)

dans laquelle

R représente de l'hydrogène, un groupe méthyle ou du fluor,

$R_1$ est de l'hydrogène ou un groupe méthyle ou forme, conjointement avec $R_2$, un groupe méthylène qui peut être substitué une ou deux fois par un radical méthyle ou une fois par un radical tertio-butyle, ou un groupe diméthylène,

$R_2$ est un groupe alkyle ayant jusqu'à 8 atomes de carbone, phényle,

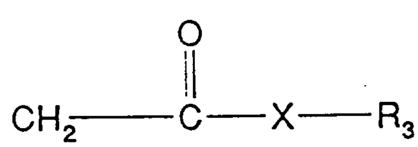

ou forme conjointement avec $R_1$ un pont tel qu'il a été décrit,

$R_3$ est un reste terpényle en $C_{10}$, un reste adamantyle ou un reste décahydronaphtyle ou représente un reste alkyle ou un reste cycloalkyle ayant chacun jusqu'à 12 atomes de carbone, à l'exception des restes méthyle et éthyle, qui porte le cas échéant un ou deux substituants aryle en $C_6$ à $C_{10}$, alkoxy en $C_1$ à $C_4$, cycloalkyle en $C_3$ à $C_{12}$ ou halogéno, les restes aryle et cycloalkyle mentionnés pouvant aussi de leur côté être substitués par des substituants alkyle en $C_1$ à $C_4$,

X est de l'oxygène ou un reste $NR_4$ dans lequel $R_4$ représente de l'hydrogène ou un radical alkyle en $C_1$ à $C_4$ ou forme conjointement avec $R_3$ un noyau pentagonal à heptagonal contenant de l'azote, qui peut éventuellement porter un ou deux substituants (alkyle en $C_1$ à $C_4$)-carbonyle ou (alkoxy en $C_1$ à $C_6$)-carbonyle,

A est un groupe méthylène ou un groupe diméthylène.

2. Dérivés d'aminoacides optiquement actifs contenant du soufre, choisis dans le groupe des exemples 1 à 25 conformément au Tableau I donné dans la description.

3. Composés de formule générale (I) suivant la revendication 1, caractérisés en ce qu'ils contiennent la séquence des aminoacides optiquement actifs contenant du soufre cystéine, homocystéine, pénicillamine, et la fonction SH est alkylée, arylée, alkoxycarbonylméthylée ou liée au groupe amino par l'intermédiaire d'un pont alkylène.

4. Procédé de production de composés de formule générale (I)

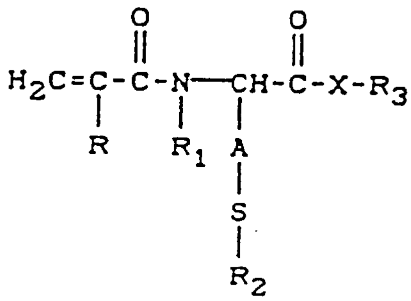

(I),

dans laquelle R, $R_1$, $R_2$, $R_3$, A et X ont la définition indiquée dans la revendication 1,

caractérisé en ce qu'on fait réagir des dérivés d'aminoacides optiquement actifs contenant du soufre de formule (II)

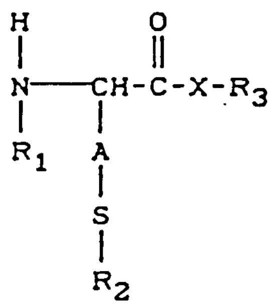

(II)

dans laquelle $R_1$, $R_2$, $R_3$, A et X ont la définition indiquée pour la formule (I),

ou leurs produits d'addition d'acides avec des dérivés d'acryloyle de formule (III)

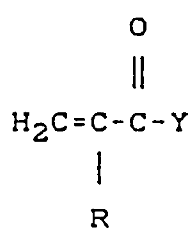

(III),

dans laquelle R a la définition indiquée pour la formule (I) et

Y représente du fluor, du chlore ou du brome ou le reste

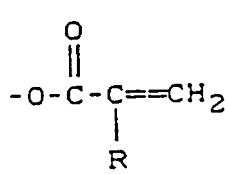

(anhydride),
le cas échéant en présence d'un accepteur d'acide, dans des solvants organiques inertes.

**5.** Polymères optiquement actifs, contenant au moins 40 moles % de motifs structuraux de formule (IV)

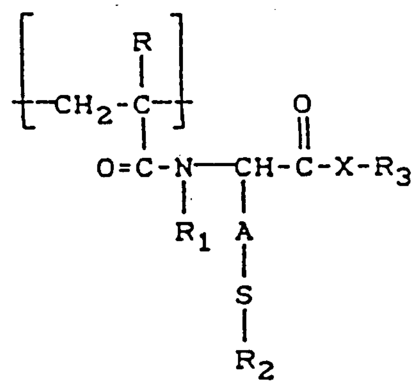

(IV)

dans laquelle

| | |
|---|---|
| R | représente un groupe méthyle ou du fluor, et |
| $R_1$, $R_2$, $R_3$, A et X | ont la définition indiquée dans la revendication 1. |

**6.** Polymères optiquement actifs suivant la revendication 5, caractérisés en ce qu'ils se présentent sous forme de produits de polymérisations en perles réticulés insolubles mais pouvant gonfler ou sous une forme liée à des supports inorganiques en fines particules.

**7.** Procédé de production de polymères en perles réticulés suivant la revendication 6, caractérisé en ce qu'on produit les dérivés de N-acryloyl-aminoacides contenant du soufre de formule générale (I) avec 0,5 à 50 moles % d'un agent de réticulation par polymérisation en suspension, le cas échéant en présence d'un générateur de radicaux et de solvants organiques inertes.

**8.** Procédé de production de polymères optiquement actifs liés à des supports inorganiques suivant la revendication 6, caractérisé en ce qu'on polymérise des composés de formule générale (I) suivant la revendication 1 en présence de phases de diols liées au gel de silice portant éventuellement un substituant (méth)acryloyle ou de phases vinyliques liées au gel de silice en présence éventuelle de solvants organiques inertes.

**9.** Utilisation de polymères optiquement actifs contenant au moins 40 moles % de motifs structuraux de formule IV suivant la revendication 6, dans laquelle

| | |
|---|---|
| R | représente de l'hydrogène, un groupe méthyle ou du fluor, |
| $R_1$, $R_2$, A et X | ont la définition indiquée dans la revendica tion 1 et |
| $R_3$ | représente un reste terpényle en $C_{10}$, un reste adamantyle ou un reste décahydronaphtyle, ou représente un reste alkyle ou un reste cycloalkyle ayant chacun jusqu'à 12 atomes de carbone, qui porte le cas échéant un ou deux substituants aryle en $C_6$ à $C_{10}$, alkoxy en $C_1$ à $C_4$, cycloalkyle en $C_3$ à $C_{12}$ ou halogéno, les restes aryle et cycloalkyle mentionnés pouvant être substitués quant à eux par un radical alkyle en $C_1$ à $C_4$, |

pour la séparation de mélanges racémiques en les antipodes optiques.

**Revendications pour les Etats contractants suivants : ES, GR**

**1.** Procédé de production de composés de formule générale (I)

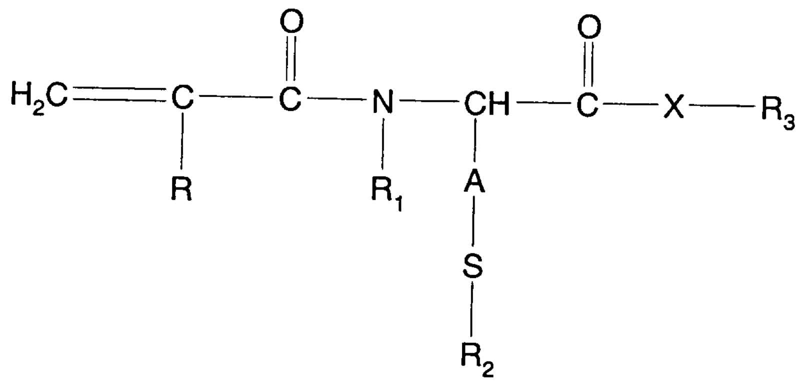

(I)

dans laquelle

R représente de l'hydrogène, un groupe méthyle ou du fluor,

$R_1$ est de l'hydrogène ou un groupe méthyle ou forme, conjointement avec $R_2$, un groupe méthylène qui peut être substitué une ou deux fois par un radical méthyle ou une fois par un radical tertio-butyle, ou un groupe diméthylène,

$R_2$ est un groupe alkyle ayant jusqu'à 8 atomes de carbone, phényle,

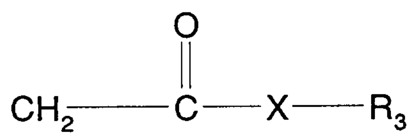

ou forme conjointement avec $R_1$ un pont tel qu'il a été décrit,

$R_3$ est un reste terpényle en $C_{10}$, un reste adamantyle ou un reste décahydronaphtyle ou représente un reste alkyle ou un reste cycloalkyle ayant chacun jusqu'à 12 atomes de carbone, à l'exception des restes méthyle et éthyle, qui porte le cas échéant un ou deux substituants aryle en $C_6$ à $C_{10}$, alkoxy en $C_1$ à $C_4$, cycloalkyle en $C_3$ à $C_{12}$ ou halogéno, les restes aryle et cycloalkyle mentionnés pouvant aussi de leur côté être substitués par des substituants alkyle en $C_1$ à $C_4$,

X est de l'oxygène ou un reste $NR_4$ dans lequel $R_4$ représente de l'hydrogène ou un radical alkyle en $C_1$ à $C_4$ ou forme conjointement avec $R_3$ un noyau pentagonal à heptagonal contenant de l'azote, qui peut éventuellement porter un ou deux substituants (alkyle en $C_1$ à $C_4$)-carbonyle ou (alkoxy en $C_1$ à $C_6$)-carbonyle,

A est un groupe méthylène ou un groupe diméthylène,

caractérisé en ce qu'on fait réagir des dérivés d'aminoacides optiquement actifs contenant du soufre de formule (II)

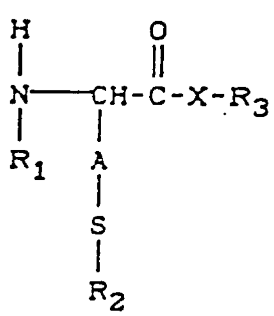

(II)

dans laquelle $R_1$, $R_2$, $R_3$, A et X ont la définition indiquée pour la formule (I),

ou leurs produits d'addition d'acides avec des dérivés d'acryloyle de formule (III)

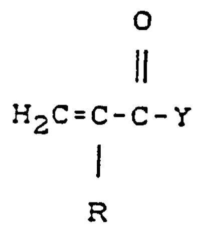

(III),

dans laquelle R a la définition indiquée pour la formule (I) et

Y représente du fluor, du chlore ou du brome ou le reste

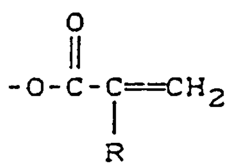

(anhydride),

le cas échéant en présence d'un accepteur d'acide, dans des solvants organiques inertes.

**2.** Procédé de production de polymères optiquement actifs, contenant au moins 40 moles % de motifs structuraux de formule IV

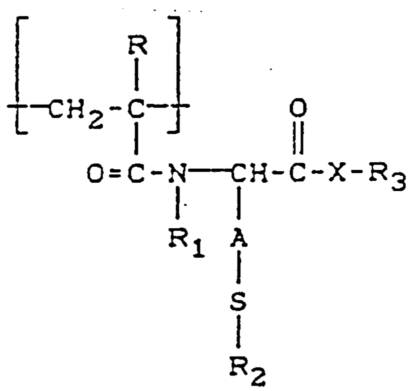

(IV)

dans laquelle

R est un groupe méthyle ou du fluor,

$R_1$, $R_2$, $R_3$, A et X ont la définition indiquée dans la revendication 1, caractérisé en ce qu'on transforme des composés de formule (I) suivant la revendication 1 d'après des procédés classiques par polymérisation ou copolymérisation des composés de formule générale (I) suivant la revendication 1 en produits de polymérisation en perles réticulés, insolubles mais gonflants ou en polymères qui sont présents sous une forme liée sur des supports inorganiques en fines particules.

**3.** Procédé de production de polymères en perles réticulés suivant la revendication 2, caractérisé en ce qu'on produit les dérivés de N-acryloylaminoacides contenant du soufre de formule générale (I) avec 0,5 à 50 moles % d'un agent de réticulation par polymérisation en suspension, le cas échéant en présence de générateurs de radicaux et de solvants organiques inertes.

**4.** Procédé de production de polymères optiquement actifs liés à des supports inorganiques suivant la revendication 2, caractérisé en ce qu'on polymérise des composés de formule générale (I) suivant la revendication 1 en présence de phases de diols liées à du gel de silice portant éventuellement des substituants (méth)acryloyle ou en présence de phases vinyliques liées à du gel de silice, en la présence éventuelle de solvants organiques inertes.

**5.** Utilisation de polymères optiquement actifs contenant au moins 40 moles % de motifs structuraux de formule IV suivant la revendication 2, dans laquelle

| | |
|---|---|
| R | représente de l'hydrogène, un groupe méthyle ou du fluor, |
| $R_1$, $R_2$, A et X | ont la définition indiquée dans la revendication 1 et |
| $R_3$ | représente un reste terpényle en $C_{10}$, un reste adamantyle ou un reste décahydronaphtyle, ou représente un reste alkyle ou un reste cycloalkyle ayant chacun jusqu'à 12 atomes de carbone, qui porte le cas échéant un ou deux substituants aryle en $C_6$ à $C_{10}$, alkoxy en $C_1$ à $C_4$, cycloalkyle en $C_3$ à $C_{12}$ ou halogéno, les restes aryle et cycloalkyle mentionnés pouvant être substitués quant à eux par un radical alkyle en $C_1$ à $C_4$, |

pour la séparation de mélanges racémiques en les antipodes optiques.